(19) Europäisches Patentamt
European Patent Office
Office européen des brevets

(11) Publication number: **0 191 562 B1**

(12) # EUROPEAN PATENT SPECIFICATION

(45) Date of publication of patent specification: **10.07.91**

(51) Int. Cl.[5]: **C07D 403/06**, A61K 31/415

(21) Application number: **86300423.0**

(22) Date of filing: **22.01.86**

The file contains technical information submitted after the application was filed and not included in this specification

(54) **Tetrahydrocarbazolone derivatives.**

(30) Priority: **23.01.85 GB 8501727**
**23.01.85 GB 8501728**

(43) Date of publication of application:
**20.08.86 Bulletin 86/34**

(45) Publication of the grant of the patent:
**10.07.91 Bulletin 91/28**

(84) Designated Contracting States:
**BE CH DE FR GB IT LI LU NL SE**

(56) References cited:
**GB-A- 2 153 821**
**US-A- 3 634 420**

(73) Proprietor: **GLAXO GROUP LIMITED**
**Clarges House 6-12 Clarges Street**
**London W1Y 8DH(GB)**

(72) Inventor: **Coates, Ian Harold**
**35, Woodlands Road Hertford**
**Hertfordshire(GB)**
Inventor: **Bell, James Angus**
**44 Hale Close Melbourne**
**Royston Hertfordshire(GB)**
Inventor: **Humber, David Cedric**
**108 Argyle Road**
**Ealing London, W13(GB)**
Inventor: **Ewan, George Blanch**
**12a Gold Hill East Chalfont St. Peter**
**Gerrard's Cross Buckinghamshire(GB)**

(74) Representative: **Marchant, James Ian et al**
**Elkington and Fife Beacon House 113 Kingsway**
**London WC2B 6PP(GB)**

**EP 0 191 562 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to heterocyclic compounds, to processes for their preparation, to pharmaceutical compositions containing them and to their medical use. In particular the invention relates to compounds which act upon certain 5- hydroxytryptamine (5-HT) receptors of the type located on terminals of primary afferent nerves.

US-A-3634420 discloses substituted amino alkylenetetrahydrocarbazoles of the following formula

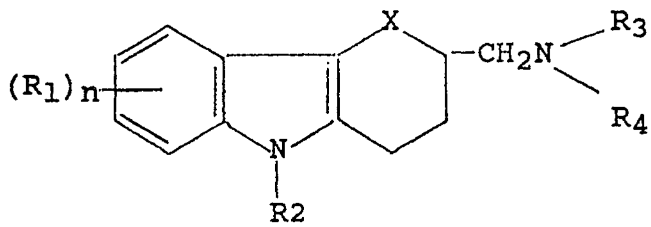

wherein $R_1$ is hydrogen or lower alkoxy;
$R_2$ is hydrogen or lower alkyl;
$R_3$ and $R_4$ are lower alkyl, hydroxy lower alkyl, or lower alkenyl, or $NR_3R_4$ is a heterocyclic ring selected from polymethyleneimino, morpholino, lower alkyl piperidino, phenyl piperidino, or mono unsaturated polymethyleneimino;
X is $CH_2$ or C=O;
n is 1 or 2.

The compounds are said to be physiologically active on the central nervous system and, in particular, to show activity as tranquilizers at non-toxic doses.

According to one aspect, the present invention provides a tetrahydrocarbazolone of the general formula (I):

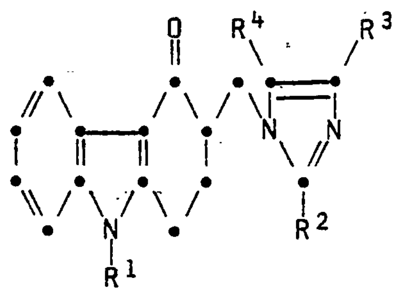

(I)

wherein

$R^1$ represents a $C_{3-7}$ cycloalkyl-$(C_{1-4})$ alkyl group or a $C_{3-10}$ alkynyl group; and one of the groups represented by $R^2$, $R^3$ and $R^4$ is a hydrogen atom or a $C_{1-6}$ alkyl, $C_{3-7}$ cycloalkyl, $C_{2-6}$ alkenyl or phenyl-$(C_{1-3})$ alkyl group and each of the other two groups, which may be the same or different, represents a hydrogen atom or a $C_{1-6}$ alkyl group; and physiologically acceptable salts and solvates (e.g. hydrates) thereof.

When the group $R^1$ in general formula (I) represents a $C_{3-7}$ cycloalkyl-$(C_{1-4})$ alkyl group, the $C_{3-7}$ cycloalkyl moiety may be, for example, a cyclopropyl, cyclobutyl, cyclopentyl, cyclohexyl or cycloheptyl group; and the $C_{1-4}$ alkyl portion may be a methyl, ethyl, propyl, prop-2-yl or butyl group. The group $R^1$ may therefore represent, e.g. a cyclopropylmethyl, cyclopentylpropyl or a cycloheptylmethyl group. When the cycloalkyl ring contains 5,6 or 7 carbon atoms it may optionally contain one or two double bonds. Examples of such groups include cyclohexenyl and cyclohexadienyl groups.

When $R^1$ represents a $C_{3-10}$ alkynyl group, this may be, for example, a 2-propynyl or 2-octynyl group. It will be understood that when $R^1$ represents a $C_{3-10}$ alkynyl group, the triple bond may not be adjacent to the nitrogen atom.

Referring to the groups represented by $R^2$,$R^3$ and $R^4$ in general formula (I), an alkyl group may be a straight chain or branched chain alkyl group, for example, a methyl, ethyl, propyl, or prop-2-yl, group;

an alkenyl group may be, for example, a propenyl group;

a phenyl-($C_{1-3}$) alkyl group may be, for example, a benzyl, phenethyl or 3-phenylpropyl group; and a cycloalkyl group may be, for example, a cyclopentyl, cyclohexyl or cycloheptyl group.

It will be appreciated that the carbon atom at the 3- position of the tetrahydrocarbazolone ring is asymmetric and may exist in the R- or S- configuration. Furthermore, it will be appreciated that depending upon the nature of the groups $R^1$, $R^2$, $R^3$ and $R^4$, centres of isomerism may occur elsewhere in the molecule. The present invention encompasses all the individual isomeric forms of the compounds of formula (I) and all mixtures thereof.

Suitable physiologically acceptable salts of the indoles of general formula (I) include acid addition salts formed with organic or inorganic acids for example, hydrochlorides, hydrobromides, sulphates, phosphates, citrates, fumarates and maleates. The solvates may, for example, be hydrates.

A preferred class of compounds represented by the general formula (I) is that wherein one of the groups represented by $R^2$, $R^3$ and $R^4$ represents a $C_{1-3}$ alkyl or $C_{3-6}$ alkenyl group and each of the other two groups, which may be the same or different, represents a hydrogen atom or a $C_{1-3}$ alkyl group. When $R^2$ represents a hydrogen atom, $R^3$ and/or $R^4$ preferably represents a $C_{1-3}$ alkyl group. When $R^2$ represents a $C_{1-3}$ alkyl group $R^3$ and $R^4$ both preferably represent hydrogen atoms.

Preferred compounds according to the invention include :

9-[(cyclopropyl)methyl]-1,2,3,9-tetrahydro-3-[(2-methyl-1H-imidazol-1-yl)methyl]-4H-carbazol-4-one;
1,2,3,9-tetrahydro-3-[(2-methyl-1H-imidazol-l-yl)methyl]-9-(prop-2-ynyl)-4H-carbazol-4-one;
9-(cyclobutylmethyl)-1,2,3,9-tetrahydro-3-[(2-methyl-1H-imidazol-1-yl)methyl]-4H-carbazol-4-one;
9-(cyclopentylmethyl)-1,2,3,9-tetrahydro-3-[(2-methyl-1H-imidazol-1-yl)methyl]-4H-carbazol-4-one;
1,2,3,9-tetrahydro-3-[(2-methyl-1H-imidazol-1-yl)methyl]-9-(2-octynyl)-4H-carbazol-4-one;
9-(3-cyclopentylpropyl)-1,2,3,9-tetrahydro-3-[(2-methyl-1H-imidazol-1-yl) methyl]-4H-carbazol-4-one; and
9-(cycloheptylmethyl)-1,2,3,9-tetrahydro-3-[(2-methyl-1H-imidazol-1-yl)methyl]-4H-carbazol-4-one
and their physiologically acceptable salts and solvates (e.g. hydrates).

It will be appreciated that the invention extends to other physiologically acceptable equivalents of the compounds according to the invention, i.e. physiologically acceptable compounds which are converted in vivo into the parent compound of formula (I).

Compounds of the invention are potent and selective antagonists of 5-HT-induced responses of the rat isolated vagus nerve preparation and thus act as potent and selective antagonists of the 'neuronal' 5-HT receptor type located on primary afferent nerves. Receptors of this type are also believed to be present in the central nervous system. 5-HT occurs widely in the neuronal pathways in the central nervous system and disturbance of these 5-HT containing pathways is known to alter behavioural syndromes such as mood, psychomotor activity, appetite and memory. It is believed that compounds which antagonise the effect of 5-HT at 5HT receptors of the type located on the terminals of primary afferent nerves will be useful in the treatment of conditions such as psychotic disorders, (e.g. schizophrenia and mania); anxiety; pain; and migraine.

Unlike existing drug treatments for these conditions the compounds of the invention, because of their high selectivity for 5-HT receptors of the type located on primary afferent nerve terminals, would not be expected to produce undesirable side effects. Thus, for example, neuroleptic drugs exhibit extrapyramidal side effects and may cause tardive dyskinesia, and benzodiazapines may cause dependence.

Compounds of formula (I) and physiologically acceptable salts and solvates thereof are useful in the treatment of a human or animal subject suffering from a condition caused by a disturbance of 'neuronal' 5HT function. Thus, for example, in the treatment of a human subject suffering from a psychotic disorder such as schizophrenia or mania; or from anxiety; pain; or migraine.

Accordingly, the invention also provides a pharmaceutical composition which comprises a least one compound selected from 3-imidazolylmethyltetrahydrocarbazolone derivatives of the general formula (I) and their physiologically acceptable salts and solvates, e.g. hydrates, adapted for use in human or veterinary medicine, and formulated for administration by any convenient route.

Such compositions may be formulated in conventional manner using one or more physiologically acceptable carriers or excipients.

Thus the compounds of the invention may be formulated for oral, buccal, parenteral, topical or rectal administration or in a form suitable for administration by inhalation or insufflation (either through the mouth or the nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl

sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by injection. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described previously, the compounds of the invention may also be formulated as a depot preparation. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

A proposed dose of the compounds of the invention for administration in man (of approximately 70kg body weight) is 0.05 to 20mg, preferably 0.1 to 10mg of the active ingredient per unit dose which could be administered, for example, 1 to 4 times per day. The dose will depend on the route of administration and the body weight of the patient. It will be appreciated that it may be necessary to make routine variations to the dosage depending on the age and weight of the patient as well as the severity of the condition to be treated.

For oral administration a unit dose will preferably contain from 0.5 to 10mg of the active ingredient. A unit dose for parenteral administration will preferably contain 0.1 to 10mg of the active ingredient.

Aerosol formulations are preferably arranged so that each metered dose or 'puff' delivered from a pressurized aerosol contains 0.2mg to 2mg, of a compound of the invention, and, each dose administered via capsules and cartridges in an insufflator or an inhaler contains 0.2 to 20mg of a compound of the invention. The overall daily dose by inhalation will be within the range 0.4 to 80mg. Administration may be several times daily, for example from 2 to 8 times, giving for example 1, 2 or 3 doses each time.

The compounds of the invention may, if desired, be administered in combination with one or more other therapeutic agents, such as anti-nauseants.

According to another aspect of the invention, compounds of general formula (I) and physiologically acceptable salts or solvates or physiologically acceptable equivalents thereof may be prepared by the general methods outlined hereinafter.

According to a first general process (A), a compound of general formula (I) or a physiologically acceptable salt or solvate or a physiologically acceptable equivalent thereof may be prepared by reacting a compound of general formula (II):

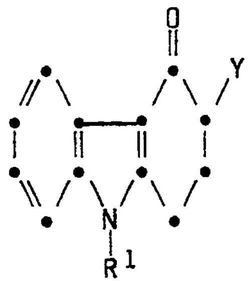

(II)

(wherein $R^1$ is as defined previously and Y represents a reactive substituent) or a protected derivative thereof with an imidazole of general formula (III):

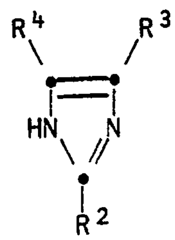

(III)

(wherein $R^2$, $R^3$ and $R^4$ are as defined previously) or a salt thereof.

Examples of compounds of formula (II) employed as starting materials in the process (A) include compounds wherein Y represents a group selected from an alkenyl group $=CH_2$ or a group of formula $CH_2Z$ where Z represents a readily displaceable atom or group such as a halogen atom, e.g. chlorine or bromine; an acyloxy group such as acetoxy, trifluoromethanesulphonyloxy, p-toluene sulphonyloxy or methanesulphonyloxy; a group $-\overset{+}{N}R^5R^6R^7\overline{X}$, (where $R^5$, $R^6$ and $R^7$, which may be the same or different each represents lower alkyl e.g. methyl; aryl e.g. phenyl; or aralkyl e.g. benzyl; or $R^5$ and $R^6$ together with the nitrogen atom to which they are attached may form a 5- to 6-membered ring e.g. a pyrrolidine ring; and X represents an anion such as a halide ion e.g. chloride, bromide or iodide); or a group $-NR^5R^6$ where $R^5$ and $R^6$ are as defined above, for example $-N(CH_3)_2$.

When Y represents the group $=CH_2$, the process may conveniently be carried out in a suitable solvent, examples of which include water; esters, e.g. ethyl acetate; ketones, e.g. acetone; or methylisobutylketone; amides, e.g. dimethylformamide; alcohols, e.g. ethanol; and ethers e.g. dioxan or tetrahydrofuran; or mixtures thereof. The process may be effected at a temperature of, for example, 20 to 100° C.

When Y represents the group $CH_2Z$, where Z is a halogen atom or an acyloxy group, the process may conveniently be carried out in a suitable solvent such as an amide, e.g. dimethylformamide; an alcohol, e.g. methanol or industrial methylated spirit; or a haloalkane, e.g. dichloromethane, and at a temperature of from -10 to 150° C, e.g. +20 to +100° C.

The reaction of a compound of formula (II) where Y represents the group $CH_2Z$ where Z is the group $-\overset{+}{N}R^5R^6R^7\overline{X}$, may conveniently be carried out in a suitable solvent such as water, an amide, e.g. dimethylformamide; a ketone, e.g. acetone; or an ether, e.g. dioxan, and at a temperature of from 20 to 150° C.

The reaction involving a compound of formula (II) where Y represents the group $-CH_2Z$, where Z is the group $-NR^5R^6$, may conveniently be carried out in a suitable solvent such as water or an alcohol, e.g. methanol, or mixtures thereof, and at a temperature of from 20 to 150° C.

According to another general process (B) a compound of formula (I) may be prepared by oxidising a compound of formula (IV):

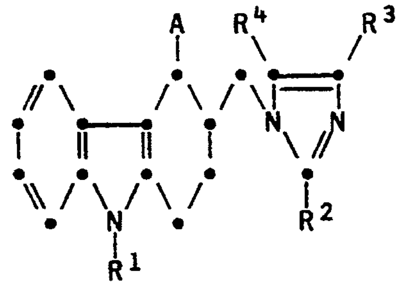

(IV)

(wherein A represents a hydrogen atom or a hydroxyl group and $R^1$, $R^2$, $R^3$ and $R^4$ are as previously defined) or a salt or a protected derivative thereof.

The oxidation process may be effected using conventional methods and the reagents and reaction conditions should be chosen such that they do not cause oxidation of the indole group. Thus, the oxidation process is preferably effected using a mild oxidising agent.

When oxidising a compound of formula (IV) in which A represents a hydrogen atom, suitable oxidising agents include quinones in the presence of water, e.g. 2,3-dichloro-5,6-dicyano-,1,4-benzoquinone or 2,3,5,6-tetrachloro-1,4-benzoquinone; selenium dioxide; a cerium (IV) oxidising reagent, such as ceric ammonium nitrate or a chromium (VI) oxidising agent, e.g. a solution of chromic acid in acetone (for example Jones' reagent) or chromium trioxide in pyridine.

When oxidising a compound of formula (IV) in which A represents a hydroxyl group, suitable oxidising agents include quinones in the presence of water, e.g. 2,3-dichloro-5,6-dicyano-1,4-benzoquinone or 2,3,5,6-tetrachloro-1,4-benzoquinone; ketones, e.g. acetone, methylethylketone or cyclohexanone, in the presence of a base e.g. aluminium t-butoxide; a chromium (VI) oxidising agent, e.g. a solution of chromic acid in acetone (for example Jones reagent) or chromium trioxide in pyridine; an N-halosuccinimide, e.g. N-chlorosuccinimide or N-bromosuccinimide; a dialkylsulphoxide e.g. dimethylsulphoxide, in the presence of an activating agent such as N,N'-dicyclohexylcarbodiimide or an acyl halide, e.g. oxalyl chloride or tosyl chloride; pyridine-sulphur trioxide complex; or a dehydrogenation catalyst such as copper chromite, zinc oxide, copper or silver.

Suitable solvents may be selected from ketones, e.g. acetone or butanone; ethers e.g. tetrahydrofuran or dioxan; amides, e.g. dimethylformamide; alcohols, e.g. methanol; hydrocarbons, e.g. benzene or toluene; halogenated hydrocarbons, e.g. dichloromethane; and water; or mixtures thereof.

The process is conveniently effected at a temperature of -70 to +50 °C. It will be understood that the choice of oxidising agent will affect the preferred reaction temperature.

According to another general process (C) a compound of general formula (I), may be prepared by alkylating a compound of general formula (V)

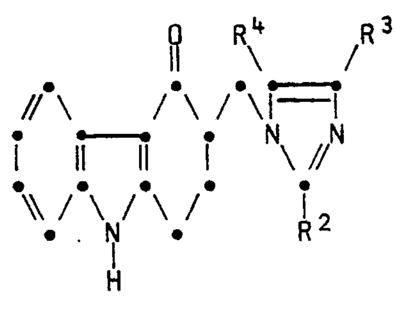

(V)

(wherein $R^2$, $R^3$ and $R^4$ are as defined previously) or a salt or protected derivative thereof.

The alkylation reaction may be effected using the appropriate alkylating agent of formula $R^1X^a$ where $X^a$ represents a leaving group such as a halogen atom, e.g. chlorine, bromine or iodine, or an acyloxy group, e.g. acetoxy, trifluoromethanesulphonyloxy, p-toluenesulphonyloxy or methanesulphonyloxy or a sulphate of formula $R^1{}_2SO_4$.

The alkylation reaction is conveniently carried out in an inert organic solvent such as an amide, e.g. dimethylformamide; an ether, e.g. tetrahydrofuran; or an aromatic hydrocarbon, e.g. toluene, preferably in the presence of a base. Suitable bases include, for example, alkali metal hydrides such as sodium hydride, alkali metal amides such as sodium amide, alkali metal carbonates such as sodium carbonate or an alkali

metal alkoxide such as sodium or potassium methoxide, ethoxide or t-butoxide. The reaction may conveniently be effected at a temperature in the range -20 to +100° C, preferably 0 to 50° C.

According to a further general process (D), a compound of general formula (I) wherein $R^1$ represents a $C_{3-7}$ cycloalkyl-($C_{1-4}$) alkyl group may be prepared by hydrogenating a compound of general formula (I) wherein $R^1$ represents a $C_{3-7}$ cycloalkenyl-($C_{1-4}$) alkyl group and/or one of $R^2$, $R^3$ and $R^4$ represents an alkenyl group, and each of the other groups are as defined previously, or a salt or a protected derivative thereof.

The hydrogenation may be effected using conventional procedures, for example by using hydrogen in the presence of a noble metal catalyst e.g. palladium, Raney nickel, platinum, platinum oxide or rhodium. The catalyst may be supported on, for example, charcoal or a homogeneous catalyst such as tris-(triphenylphosphine) rhodium chloride may be used. The hydrogenation will generally be effected in a solvent such as an alcohol, e.g. ethanol; an amide, e.g. dimethylformamide; an ether, e.g. dioxan; or an ester, e.g. ethyl acetate, and at a temperature in the range -20 to 100° C, preferably 0 to 50° C.

It should be appreciated that in some of the above transformations it may be necessary or desirable to protect any sensitive groups in the compound to avoid undesirable side reactions. The protecting groups used in the preparation of compounds of formula (I) are desirably groups which may be readily split off at a suitable stage in the reaction sequence, conveniently at the last stage. For example, during any of the reaction sequences described above, it may be necessary to protect the keto group, for example, as a ketal or a thioketal.

Compounds of general formula (I) may thus be prepared according to another general process (E), which comprises removal of any protecting groups from a protected form of a compound of formula (I). Deprotection may be effected using conventional techniques such as those described in 'Protective Groups in Organic Chemistry' Ed.J.F.W McOmie (Plenum Press, 1973). Thus, a ketal such as an alkyleneketal group may be removed by treatment with a mineral acid such as hydrochloric acid. The thioketal group may be cleaved by treatment with a mercuric salt, e.g. mercuric chloride, in a suitable solvent, such as ethanol.

The compounds of formula (I) may be converted into their physiologically acceptable salts according to conventional methods. Thus, for example, the free base of general formula (I) may be treated with an appropriate acid, preferably with an equivalent amount in a suitable solvent (e.g. aqueous ethanol).

Physiologically acceptable equivalents of a compound of formula (I) may be prepared according to conventional methods.

Individual enantiomers of the compounds of the invention may be obtained by resolution of a mixture of enantiomers (e.g a racemic mixture) using conventional means, such as an optically active resolving acid; see for example 'Stereochemistry of Carbon Compounds' by E.L.Eliel (McGraw Hill 1962) and 'Tables of Resolving Agents' by S. H. Wilen.

Examples of optically active resolving acids that may be used to form salts with the racemic compounds include the (R) and (S) forms of organic carboxylic and sulphonic acids such as tartaric acid, dip-toluoyltartartic acid, camphorsulphonic acid and lactic acid. The resulting mixture of isomeric salts may be separated, for example, by fractional crystallisation, into the diastereoisomers and if desired, the required optically active isomer may be converted into the free base.

The methods indicated above for preparing the compounds of the invention can be used as the last main step in the preparative sequence. The same general methods can be used for the introduction of the desired groups at an intermediate stage in the stepwise formation of the required compound, and it will be appreciated that these general methods can be combined in different ways in such multi-stage processes. The sequence of the reactions in multi-stage processes should of course be chosen so that the reaction conditions used do not affect groups in the molecule which are desired in the final product.

The starting materials of formula (II) wherein Y represents the group $=CH_2$ may be prepared from compounds of formula (II) where Y represents the group $CH_2\overset{+}{N}R^5R^6R^7X^-$ by reaction with a base in a suitable solvent. Examples of bases include alkali metal hydroxides, e.g. potassium hydroxide or alkali metal carbonates or hydrogen carbonates e.g. sodium hydrogen carbonate.

The quaternary salts may be formed from the corresponding tertiary amine by reaction with an alkylating agent such as methyl iodide or dimethyl sulphate, if preferred in a suitable solvent, e.g. dimethylformamide. The tertiary amine may be prepared by reaction of a tetrahydrocarbazolone of general formula (VI):

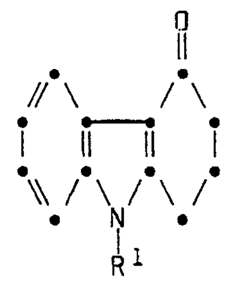

(VI)

with formaldehyde and the corresponding secondary amine, if desired in a suitable solvent such as an alcohol, e.g. ethanol.

Compounds of general formula (VI) may be prepared for example, by the method described by H. Iida et al., in J.Org.Chem. (1980) Vol 45, No.15, pages 2938-2942.

The starting materials of general formula (II) where Y represents $-CH_2Z$ where Z is a halogen atom or an acyloxy group may be prepared from the corresponding hydroxymethyl derivative of general formula (VII):

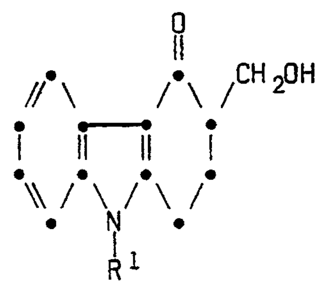

(VII)

which may be obtained by reacting the tetrahydrocarbazolone of general formula (VI) with formaldehyde, preferably in a suitable solvent such as an alcohol, e.g. ethanol, and preferably in the presence of a base.

Thus, the compounds where Z is a halogen atom may be obtained by reacting a compound of formula (VII) with a halogenating agent such as a phosphorus trihalide, e.g. phosphorus trichloride.

The compounds where Z is an acyloxy group may be prepared by reacting a compound of formula (VII) with an appropriate acylating agent such as an anhydride or a sulphonyl halide such as sulphonyl chloride.

Compounds of formula (II) where Y represents $-CH_2Z$ where Z is a halogen atom may also be prepared by reacting a compound of formula (II) where Y represents the group $=CH_2$ with the appropriate hydrogen halide, e.g. hydrogen chloride, conveniently in a suitable solvent such as an ether, e.g. diethyl ether.

Compounds of general formula (IV) may be prepared by reacting a compound of formula (VIII):

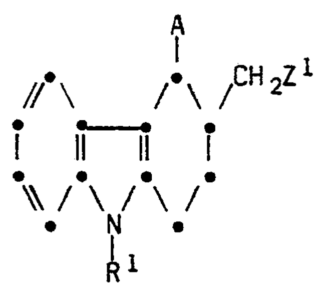

(VIII)

(wherein $R^1$ and A are as defined previously and $Z^1$ is a readily displaceable atom or group such as a halogen atom, an acyloxy group or the group $-NR^5R^6R^7X$ as previously defined for $Z^1$) with an imidazole of formula (III) according to the method of process (A) described herein.

Compounds of formula (VIII) may be prepared by reducing compounds of formula (II) using for example lithium aluminium hydride or sodium borohydride.

Compounds of formula (VIII) wherein A represents a hydrogen atom may also be prepared by reacting a compound of formula (VIII) wherein A represents a hydroxyl group with a tosyl halide (e.g. tosyl chloride)

and then reducing the resulting tosylate with lithium aluminium hydride.

Compounds of formula (IV) are novel compounds, and as such provide a further feature of the invention. The following examples illustrate the invention. All temperatures are in °C. 'Hyflo' is a filtration aid. Where indicated, solutions were dried over $Na_2SO_4$ and solids were dried in vacuo over $P_2O_5$ at 50° overnight. Chromatography was carried out using the technique described by W.C. Still et al (J. Or . Chem., 1978, 43, 2923-2925), on kieselgel 9385. The following abbreviations define the eluent used for column chromatography and t.l.c.

(A) Methylene chloride-ethanol-0.88 ammonia 100:10:1
(B) Methylene chloride-ethanol-0.88 ammonia 100: 9:1
(C) Methylene chloride-ethanol-0.88 ammonia 200:10:1
(D) Methylene chloride-ethanol-0.88 ammonia 400:10:1

PREPARATION 1

2,3,4,9-Tetrahydro-N,N,N-trimethyl-4-oxo-1H-carbazole-3-methanaminium iodide.

A solution of 3-[(dimethylamino)methyl]-1,2,3,9-tetrahydro-4H-carbazol-4-one (0.53g) in iodomethane (15ml) was heated under reflux for 5h and evaporated to dryness, giving the title compound as a white solid (0.84g) m.p. 202-205°.

PREPARATION 2

1,2,3,9-Tetrahydro-3-[(2-methyl-1H-imidazol-1-yl)methyl]-4H-carbazol-4 -one

A suspension of the product from Preparation 1 (6.6g) and 2-methyl imidazole (17.0g) in dry dimethylformamide (75mℓ) was stirred at 100° under nitrogen for 17.25h then cooled in ice to deposit a solid. This material was purified by washing successively with dimethylformamide (2x7mℓ) and dry ether (3x15mℓ)followed by column chromatography (A) to give the title compound (1.6g) m.p. 235-238° dec.

EXAMPLE 1

9-[(Cyclopropyl)methyl]-1,2,3,9-tetrahydro-3-[(2-methyl-1H-imidazol-1-yl)methyl]-4H-carbazol-4-one,hydrochloride

Sodium hydride (80% disp. in oil 0.075g) was added to a solution of the product from Preparation 2 (0.7g) in dimethylformamide (10mℓ) and stirred for 15 min. (Bromomethyl)cyclopropane (0.33g) was added and the solution maintained at 40° for 4h. before partitioning between aqueous sodium carbonate (2N, 100mℓ) and ethyl acetate (2x50mℓ). The combined organic extracts were washed with water (50mℓ), dried and evaporated in vacuo to give an oil which was purified by column chromatography (B) to give pure free base. A solution of the free base in ethanol (15mℓ) was acidified with ethereal hydrogen chloride and diluted with dry ether to precipitate the title compound (0.4g) m.p. 120-130°; N.m.r. δ ($CD_3SOCD_3$) 0.4-0.6 (4H,m,cyclopropyl-$CH_2CH_2$-), 1.2-1.3 (1H,m,cyclopropyl-CH-) 2.7 (3H,s,-$CH_3$), 4.29 and 4.69 (2H, ABX,$CHCH_2N$) and 7.2-8.1 (6H,m,aromatic).

Analysis Found: C,63.6;H,6.55;N,10.5.
$C_{21}H_{23}N_3O.HCl.1.5H_2O$ requires C,63.5;H,6.8;N,10.6%.

EXAMPLE 2

1,2,3,9-Tetrahydro-3-[(2-methyl-1H-imidazol-1-yl)methyl]9-(2-propynyl -4H-carbazol-4-one,oxalate

Sodium hydride (80% disp. in oil, 0.075g) was added to a solution of the product from Preparation 2 (0.7g) in dimethylformamide (10mℓ)and stirred for 15 min. Propargyl bromide (0.44g) was added and the solution maintained at 40° for 5h before partitioning between aqueous sodium carbonate (2N, 100mℓ) and ethyl acetate (2 x 50mℓ). The combined organic layers were washed with water (2 x 50mℓ), dried and

evaporated in vacuo to give an oil which was purified by chromatography (B) to give a solid. This solid was dissolved in ethanol (10mℓ)and a solution of oxalic acid (0.14g) in methanol (5mℓ) added. Addition of dry ether precipitated the title compound (0.35g) m.p. 237-238°; N.m.r. δ ($CD_3SOCD_3$), 2.60 (3H,s,$CH_3$), 3.47 (1H,t,C≡C-H), 4.25 and 4.65 (2H, ABX $CHCH_2N$), 5.19 (2H,d,$NCH_2C≡C$), and 7.2-8.1 (6H,m,aromatic).

Analysis Found: C,64.0; H,5.0; N,10.0.

$C_{20}H_{19}N_3O.C_2H_2O_4.0.3H_2O$ requires C,63.9; H,5.1; N,10.2%.

EXAMPLE 3

**9-(Cyclobutylmethyl)-1,2,3,9-tetrahydro-3-[(2-methyl-1H-imidazol-1-yl)methyl] -4H-carbazol-4-one maleate**

A solution of 1,2,3,9-tetrahydro-3-[(2-methyl-1H-imidazol-1-yl)methyl]-4H-carbazol-4-one (1.00g) in dry dimethylformamide (10mℓ) was added dropwise under nitrogen to a stirred suspension of sodium hydride (78% in oil; 0.12g) in dry dimethylformamide (5mℓ). The mixture was stirred for 1.5h and a solution of cyclobutanemethanol (4-methylbenzenesulphonate) (2.35g) in dry dimethylformamide (5mℓ) was added. The solution was heated to 50°, stirred for 3.25h, allowed to cool and concentrated in vacuo to ca 10mℓ. The residual solution was poured onto water (100mℓ) and extracted with ethyl acetate (3x100mℓ). The combined extracts were washed with water (5x70mℓ) and brine (70mℓ), dried ($Na_2SO_4$) and concentrated in vacuo.

Flash chromatography (C) of the residual gum afforded a foam (0.67g). A portion of this material (0.47g) was dissolved in boililng absolute ethanol (2mℓ) and a solution of maleic acid (0.175g) in refluxing absolute ethanol (1mℓ)was added. The stirred solution was diluted with dry ether (50mℓ) and the resultant solid filtered off, washed with dry ether (3x15mℓ) and dried in vacuo at 60° for 21h to give the title compound as a solid (0.54g) m.p. 125.5° - 127°.

Analysis Found: C,66.3; H,6.3; N,8.8.

$C_{22}H_{25}N_3O.C_4H_4O_4.0.58H_2O$ requires C,65.9; H,6.4; N,8.9%.

N.m.r. δ(DMSO) includes 1.7-2.2 (8H,m,cyclobutane and H-2 ax); 2.65 (3H,s,Ar-$CH_3$); and 4.2-4.75 (4H,m,indole N-$CH_2$ and imidazole N-$CH_2$).

The following compounds were prepared according to the method of Example 3, using the reaction conditions given in Table 1 hereinafter.

EXAMPLE 4

**9-(Cyclopentylmethyl)-1,2,3,9-tetrahydro-3-[(2-methyl-1H-imidazol-1-yl)methyl] -4H-carbazol-4-one maleate**

m.p. 107°-108°.

Analysis Found: C,65.2; H,6.55; N,8.2.

$C_{23}H_{2/}N_3O.C_4H_4O_4.H_2O$ requires C,65.4; H,6.7; N,8.5%.

N.m.r. δ(DMSO) includes 1.2-2.45 (11H,m,cyclopentane and H-2); 2.65 (3H,s,Ar-$CH_3$); 4.17 (2H,d,N-$CH_2$-cyclopentane); and 4.27,4.66 (2H,ABX, imidazole $NCH_2$).

EXAMPLE 5

**1,2,3,9-Tetrahydro-3-[(2-methyl-1H-imidazol-1-yl)methyl]-9-(2-octynyl)-4H-carbazol-4-one, maleate**

m.p. 115-116°.

Analysis Found: C,69.2; H,6.6; N,8.3.

$C_{25}H_{29}N_3O.C_4H_4O_4$ requires C,69.2; H,6.6; N,8.3%.

N.m.r. δ($CDCl_3$) includes 0.85 (3H,t,≡C($CH_2$)$_4$$CH_3$); 1.2-1.52 (6H,m,≡C-$CH_2$<u>$CH_2$</u><u>$CH_2$</u><u>$CH_2$</u>$CH_3$); 2.8 (3H,s,Ar-$CH_3$); 4.53 (2H,ABX,CH$CH_2$N); and 4.80 (2H,t,N$CH_2$C≡).

EXAMPLE 6

**9-(3-Cyclopentylpropyl)-1,2,3,9-tetrahydro-3-[(2-methyl-1H-imidazol-1-yl) methyl]-4H-carbazol-4-one, maleate**

m.p. 119-120.5°.

Analysis Found: C,69.1; H,7.2; N,8.3.

$C_{25}H_{31}N_3O_4.C_4H_4O_4$ requires C,68.9; H,7.0; N,8.3%.

N.m.r. δ($CDCl_3$) includes 0.95-1.85 (13H, cyclopentane and $CH_2CH_2$CH); 2.80 (3H,s,Ar-$CH_3$); 4.08 (2H,t,N$CH_2$$CH_2$); and 4.55 (2H,ABx, N$CH_2$CH).

EXAMPLE 7

**9-(Cycloheptylmethyl)-1,2,3,9-tetrahydro-3-[(2-methyl-1H-imidazol-1-yl)methyl]4H-carbazol-4-one, maleate**

Analysis Found: C,66.6; H,7.0; N,7.7.

$C_{25}H_{31}N_3O.1.5C_4H_4O_4$ requires C,66.1; H,6.6; N,7.5%.

N.m.r. δ($CDCl_3$) includes 1.15-1.75 (12H,m,cycloheptane $CH_2$); 2.80 (3H,s,Ar-$CH_3$); 3.92 (2H,d,N$CH_2$-cycloheptane); and 4.56 (2H, ABX,N$CH_2$CH).

TABLE 1

| | ALKYLATION | | | | | | | | SALT FORMATION (MALEATE) | | |
|---|---|---|---|---|---|---|---|---|---|---|---|
| Ex. No. | Starting material (g) | NaH (g) | Alkylating agent: Structure | Alkylating agent: Weight (g) | Reaction time (h) | Reaction temp. (°C) | Eluent | Yield of base (g) | Wt. of base (g) | Wt. of acid (g) | Yield (g) |
| 4 | 1.0 | 0.12 | 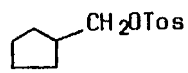 | 2.0 | 0.75+4.0 | 55 | C | 1.0 | 0.4 | 0.32 | 1.08 |
| 5 | 0.5 | 0.062 | $BrCH_2C{\equiv}CC_5H_{11}$ | 0.378 | 0.25+6<br>60 | RT<br>4-5 | D | 0.590 | 0.590 | 0.194 | 0.648 |
| 6 | 0.5 | 0.062 | 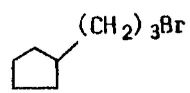 | 0.378 | 0.25+24 | RT | C | 0.615 | 0.615 | 0.203 | 0.507 |
| 7 | 0.2 | 0.024 | 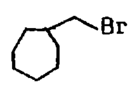 | 0.151 | 0.25+20 | RT | C | 0.136 | 0.136 | 0.045 | 0.095 |

Tos = p-toluenesulphonyl

RT = room temperature

The following examples illustrate pharmaceutical formulations according to the invention.

TABLETS FOR ORAL ADMINISTRATION

Tablets may be prepared by the normal methods such as direct compression or wet granulation.

The tablets may be film coated with suitable film forming materials, such as hydroxypropyl methylcellulose, using standard techniques. Alternatively the tablets may be sugar ccated.

Direct Compression

| Tablet | mg/tablet |
|---|---|
| Active Ingredient | 8.0 |
| Lactose NF* | 89.5 |
| Croscarmellose Sodium NF | 2.0 |
| Magnesium Stearate BP | 0.5 |
| Compression weight | 100.0 |

* of a grade suitable for direct compression.

The active ingredient is passed through a 60 mesh sieve, blended with the lactose, croscarmellose sodium and magnesium stearate. The resultant mix is compressed into tablets using 5.5mm diameter punches.

Tablets of other strengths may be prepared by altering the ratio of active ingredient to lactose or the compression weight and using punches to suit.

Injection

| | mg/ml |
|---|---|
| Active Ingredient | 2.0 |
| Sodium Chloride BP | as required |
| Water for Injection BP to | 1.0ml |

Sodium chloride may be added to adjust the tonicity of the solution and the pH may be adjusted, using acid or alkali, to that of optimum stability and/or to facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

**Claims**

**1.** Compounds of the general formula (I)

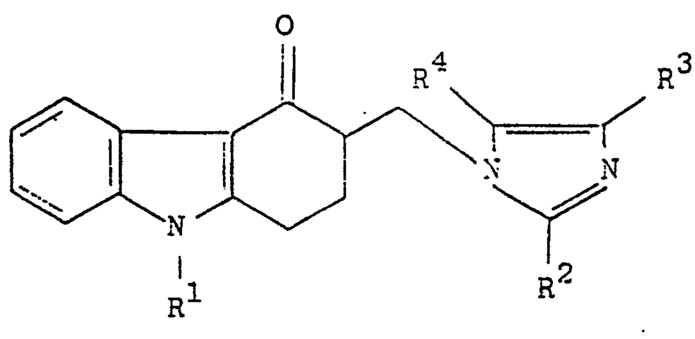

(I)

wherein

$R^1$ represents a $C_{3-7}$ cycloalkyl-($C_{1-4}$)alkyl group or a $C_{3-10}$ alkynyl group; and one of the groups represented by $R^2$, $R^3$ and $R^4$ is a hydrogen atom or a $C_{1-6}$ alkyl, $C_{3-7}$ cycloalkyl, $C_{2-6}$ alkenyl or phenyl-($C_{1-3}$) alkyl group and each of the other two groups, which may be the same or different, represents a hydrogen atom or a $C_{1-6}$ alkyl group;

and physiologically acceptable salts and solvates thereof.

**2.** Compounds as claimed in claim 1 in which one of the groups represented $R^2$, $R^3$ and $R^4$ represents a $C_{1-3}$ alkyl or $C_{3-6}$ alkenyl group and each of the other two groups, which may be the same or different, represents a hydrogen atom or a $C_{1-3}$ alkyl group.

**3.** Compounds as claimed in claim 1 in which $R^2$ represents a hydrogen atom and $R^3$ and/or $R^4$ represents a $C_{1-3}$ alkyl group.

**4.** Compounds as claimed in claim 1 in which $R^2$ represents a $C_{1-3}$ alkyl group and $R^3$ and $R^4$ both represent hydrogen atoms.

**5.** 9-[(Cyclopropyl)methyl]-1,2,3,9-tetrahydro-3-[(2-methyl-1H-imidazol-1-yl)methyl)-4H-carbazol-4-one;
1,2,3,9-tetrahydro-3-[(2-methyl-1H-imidazol-1-yl)methyl]-9-(2-propynyl)-4H-carbazol-4-one;
and physiologically acceptable salts and solvates thereof.

**6.** A process for the preparation of a compound of general formula (I) as defined in any of claims 1 to 5 or a physiologically acceptable salt or solvate thereof, which process comprises:

(A) reacting a compound of general formula (II)

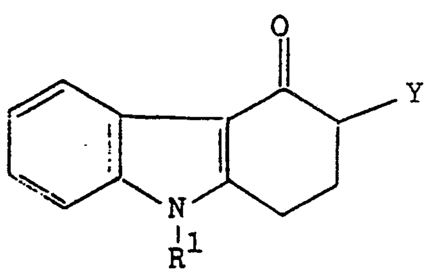

(II)

(in which $R^1$ is as defined in claim 1 and Y represents a reactive substituent) or a protected derivative thereof with an imidazole of general formula (III)

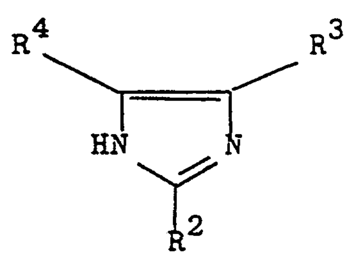

(III)

(in which $R^2$, $R^3$ and $R^4$ are as defined in claim 1) or a salt thereof with subsequent removal of any protecting group which is present; or

(B) oxidizing a compound of formula (IV)

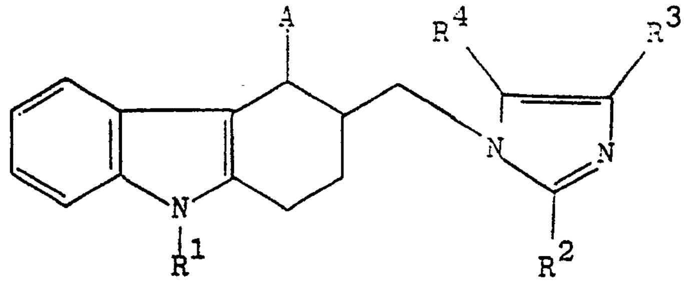

(IV)

(in which A represents a hydrogen atom or a hydroxyl group and $R^1$, $R^2$, $R^3$ and $R^4$ are as defined in claim 1) or a salt or a protected derivative thereof with subsequent removal of any protecting group which is present; or

(C) alkylating a compound of general formula (V)

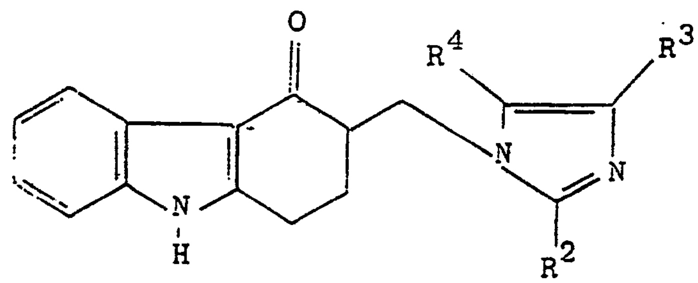

(V)

(in which $R^2$, $R^3$ and $R^4$ are as defined in claim 1) or a salt or protected derivative thereof with subsequent removal of any protecting group which is present; or

(D) for the production of a compound of general formula (I) in which $R^1$ represents a $C_{3-7}$ cycloalkyl-($C_{1-4}$) alkyl group, hydrogenating a compound of general formula (I) in which $R^1$ represents a $C_{3-7}$ cycloalkenyl-($C_{1-4}$) alkyl group and/or one of $R^2$, $R^3$ and $R^4$ represents an alkenyl group and each of the other groups are as defined in claim 1, or a salt or a protected derivative thereof with subsequent removal of any protecting group which is present; or

(E) removing a protecting group or groups from a protected form of a compound of formula (I);

and when a compound of formula (I) is obtained as a mixture of enantiomers, optionally resolving the mixture to obtain the desired enantiomer;

and/or where the compound of formula (I) is in the form of a free base, optionally converting the free base into a salt.

7. A process as claimed in claim 6 (A) in which Y represents an alkenyl group $=CH_2$ or a group of formula $CH_2Z$ where Z represents a readily displaceable atom or group.

8. A pharmaceutical composition comprising at least one compound of general formula (I) as defined in any of claims 1 to 5 or a physiologically acceptable salt or solvate thereof together with at least one physiologically acceptable carrier or excipient.

**Revendications**

1. Composés de formule générale (I)

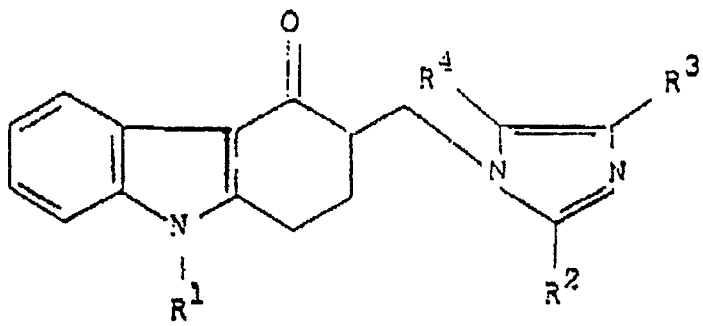

(I)

dans laquelle $R^1$ représente un groupe (cycloalkyle en $C_{3-7}$)alkyle en $C_{1-4}$ ou un groupe alcynyle en $C_{3-10}$ ; et l'un des groupes représentés par $R^2$, $R^3$ et $R^4$ représente un atome d'hydrogène ou un groupe alkyle en $C_{1-6}$, cycloalkyle en $C_{3-7}$, alcényle on $C_{2-6}$ ou phénylalkyle on $C_{1-3}$ et chacun des deux autres groupes, qui peuvent être identiques ou différents, représente un atome d'hydrogène ou un groupe alkyle en $C_{1-6}$ ;
et leurs sels et solvates physiologiquement acceptables.

2. Composés selon la revendication 1, dans lesquels l'un des groupes représentés par $R^2$, $R^3$ et $R^4$ représente un groupe alkyle en $C_{1-3}$ ou alcényle en $C_{3-6}$ et chacun des deux autres groupes, qui peuvent être identiques ou différents, représente un atome d'hydrogène ou un groupe alkyle en $C_{1-3}$.

3. Composés selon la revendication 1, dans lesquels $R^2$ représente un atome d'hydrogène et $R^3$ et/ou $R^4$ représentent un groupe alkyle en $C_{1-3}$.

4. Composés selon la revendication 1, dans lesquels $R^2$ représente un groupe alkyle en $C_{1-3}$ et $R^3$ et $R^4$ représentent tous les deux des atomes d'hydrogène.

5. 9-((cyclopropyl)méthyl)-1,2,3,9-tétrahydro-3-((2-méthyl-1H-imidazol-1-yl)méthyl)-4H-carbazol-4-one ;
1,2,3,9-tétrahydro-3-((2-méthyl-1H-imidazol-1-yl)méthyl)-9-(2-propynyl)-4H-carbazol-4-one ;
et leurs sels et solvates physiologiquement acceptables.

6. Procédé pour la préparation d'un composé de formule générale (I) selon l'une quelconque des revendications 1 à 5 ou d'un de ses sels ou solvates physiologiquement acceptables, qui comprend ;
(A) la réaction d'un composé de formule générale (II)

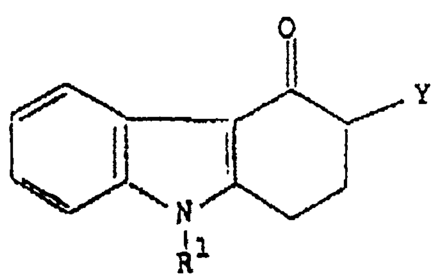

(II)

(dans laquelle $R^1$ est tel que défini dans la revendication 1 et Y représente un substituant réactif) ou d'un de ses dérivés protégés, avec un imidazole de formule générale (III)

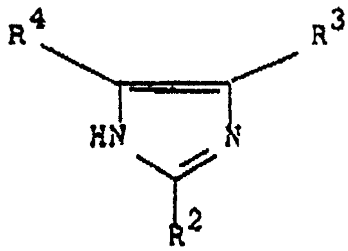

(III)

(dans laquelle $R^2$, $R^3$ et $R^4$ sont tels que définis dans la revendication 1), ou un de ses sels, avec l'élimination ultérieure de n'importe quel groupe protecteur qui est présent ; ou
(B)l'oxydation d'un composé de formule (IV)

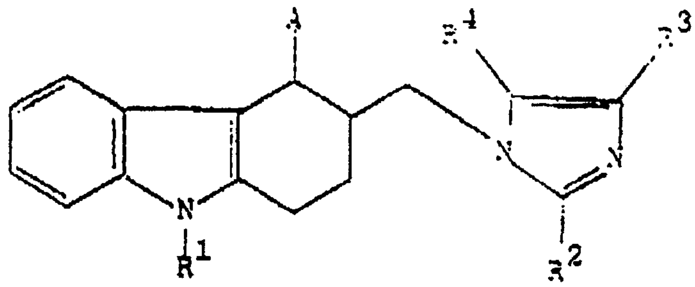

(IV)

(dans laquelle A représente un atome d'hydrogène ou un groupe hydroxyle et $R^1$, $R^2$, $R^3$ et $R^4$ sont tels que définis dans la revendication 1) ou d'un de ses sels ou de ses dérivés protégés, avec l'élimination ultérieure de n'importe quel groupe protecteur qui est présent ; ou
(C) l'alkylation d'un composé de formule (V)

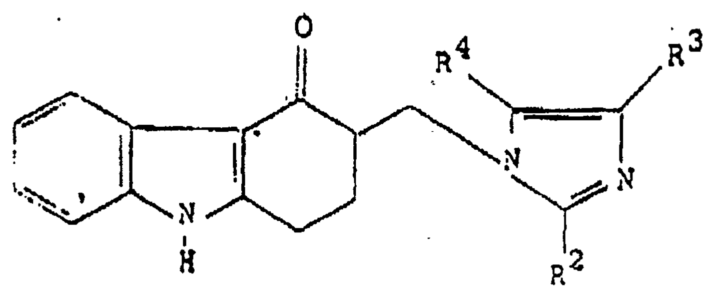

(V)

(dans laquelle $R^2$, $R^3$ et $R^4$ sont tels que définis dans la revendication 1) ou d'un de ses sels ou de ses dérivés protégés, avec l'élimination ultérieure de n'importe quel groupe protecteur qui est présent ; ou
(D) pour la production d'un composé de formule générale (I) dans laquelle $R^1$ représente un groupe (cycloalkyle en $C_{3-7}$)alkyle en $C_{1-4}$, l'hydrogénation d'un composé de formule générale (I) dans laquelle $R^1$ représente un groupe (cycloalcényle en $C_{3-7}$)alkyle en $C_{1-4}$ et/ou l'un des $R^2$, $R^3$ et $R^4$ représente un groupe alcényle et chacun des autres groupes est tel que défini dans la revendication 1, ou d'un de ses sels ou de ses dérivés protégés, avec l'élimination ultérieure de n'importe quel groupe protecteur qui est présent ; ou
(E) l'élimination d'un groupe ou de groupe(s) protecteur(s) d'une forme protégée d'un composé de formule (I) ;
et lorsque l'on obtient un composé de formule (I) sous la forme d'un mélange d'énantiomères, la résolution facultative du mélange pour obtenir l'énantiomère souhaité ;
et/ou lorsque le composé de formule (I) est sous la forme d'une base libre, la conversion éventuelle de la base libre en un sel.

7. Procédé selon la revendication 6 (A), dans lequel Y représente un groupe alcényle $=CH_2$ ou un groupe

de formule $CH_2Z$ où Z représente un groupe ou un atome facilement déplaçable.

8. Composition pharmaceutique qui comprend au moins un composé de formule générale (I) tel que défini selon l'une quelconque des revendications 1 à 5 ou un de ses sels ou solvates physiologiquement acceptables, conjointement avec au moins un véhicule ou excipient physiologiquement acceptable.

**Ansprüche**

1. Verbindungen der allgemeinen Formel (I)

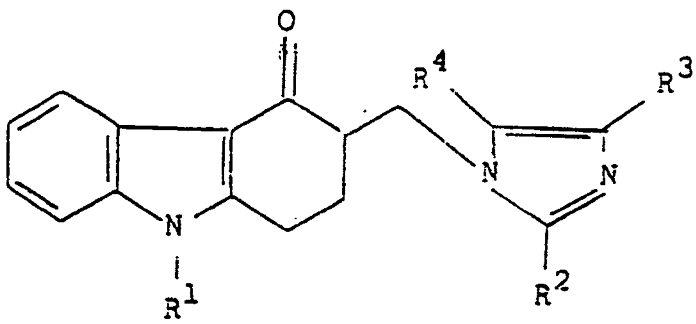

(I)

worin $R^1$ eine $C_{3-7}$-Cycloalkyl-($C_{1-4}$)-alkylgruppe oder eine $C_{3-10}$-Alkinylgruppe bedeutet; und eine der Gruppen, die durch $R^2$, $R^3$ und $R^4$ dargestellt werden, ein Wasserstoffatom oder eine $C_{1-6}$-Alkyl-, $C_{3-7}$-Cycloalkyl-, $C_{2-6}$-Alkenyl- oder Phenyl-($C_{1-3}$)-alkylgruppe bedeutet und jede der beiden anderen Gruppen, die gleich oder unterschiedlich sein können, ein Wasserstoffatom oder eine $C_{1-6}$-Alkylgruppe bedeuten; und die physiologisch annehmbaren Salze und Solvate davon.

2. Verbindung nach Anspruch 1, dadurch **gekennzeichnet**, daß eine der Gruppen, die durch $R^2$, $R^3$ und $R^4$ dargestellt werden, eine $C_{1-3}$-Alkyl- oder $C_{3-6}$-Alkenylgruppe bedeutet und jede der beiden anderen Gruppen, die gleich oder unterschiedlich sein können, ein Wasserstoffatom oder eine $C_{1-3}$-Alkylgruppe bedeuten.

3. Verbindung nach Anspruch 1, dadurch **gekennzeichnet**, daß $R^2$ ein Wasserstoffatom und $R^3$ und $R^4$ eine $C_{1-3}$-Alkylgruppe bedeutet.

4. Verbindung nach Anspruch 1, dadurch **gekennzeichnet**, daß $R^2$eine $C_{1-3}$-Alkylgruppe und $R^3$ und $R^4$ beide Wasserstoffatome bedeuten.

5. 9-[(Cyclopropyl)-methyl]-1,2,3,9-tetrahydro-3-[(2-methyl-1H-imidazol-1-yl)-methyl]-4H-carbazol-4-on; 1,2,3,9-Tetrahydro-3-[(2-methyl-1H-imidazol-1-yl)-methyl]-9-(2-propinyl)-4H-carbazol-4-on; und die physiologisch annehmbaren Salze und Solvate davon.

6. Verfahren zur Herstellung einer Verbindung der allgemeinen Formel (I) nach irgendeinem der Ansprüche 1 bis 5 oder eines physiologisch annehmbaren Salzes oder Solvates davon, dadurch **gekennzeichnet**, daß
(A) eine Verbindung der allgemeinen Formel (II)

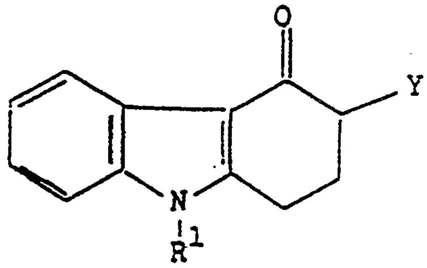

(II)

(worin $R^1$ die in Anspruch 1 gegebene Definition bedeutet und Y ein reaktiver Substituent bedeutet) oder ein geschütztes Derivat davon mit einem Imidazol der allgemeinen Formel (III)

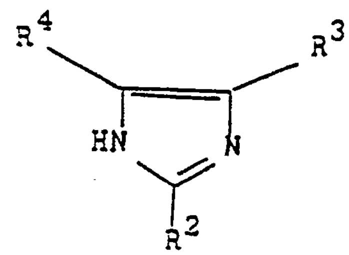

(III)

(worin $R^2$, $R^3$ und $R^4$ die in Anspruch 1 gegebene Definition besitzen) oder ein Salz davon umgesetzt wird und irgendeine vorhandene Schutzgruppe anschließend entfernt wird; oder
(B) eine Verbindung der Formel (IV)

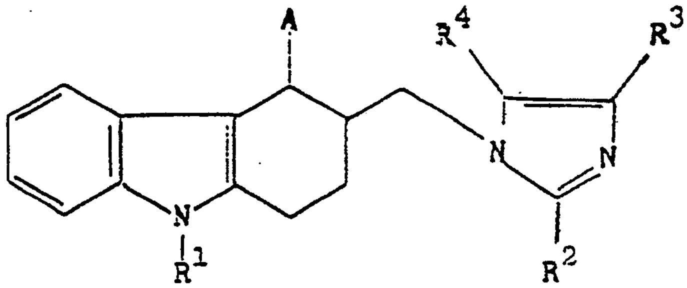

(IV)

(worin A ein Wasserstoffatom oder eine Hydroxylgruppe bedeutet und $R^1$, $R^2$, $R^3$ und $R^4$ die in Anspruch 1 gegebenen Definitionen besitzen) oder ein Salz oder ein geschütztes Derivat davon oxidiert wird und anschließend irgendeine vorhandene Schutzgruppe entfernt wird; oder
(C) eine Verbindung der allgemeinen Formel (V)

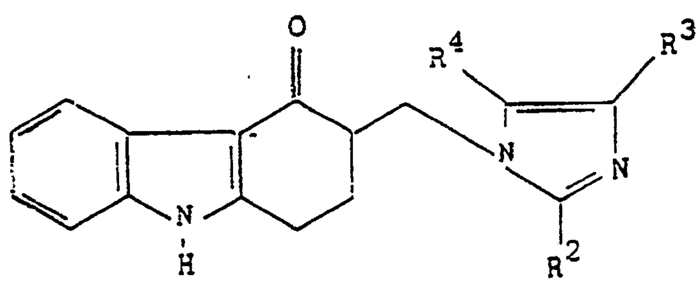

(V)

(worin $R^2$, $R^3$ und $R^4$ die in Anspruch 1 gegebenen Definitionen besitzen) oder ein Salz oder geschütztes Derivat davon alkyliert wird und anschließend irgendeine vorhandene Schutzgruppe entfernt wird; oder
(D) für die Herstellung einer Verbindung der allgemeinen Formel (I), worin $R^1$ eine $C_{3-7}$-Cycloalkyl-$(C_{1-4})$-alkylgruppe bedeutet, eine Verbindung der allgemeinen Formel (I), worin $R^1$ eine $C_{3-7}$-Cycloalkenyl-$(C_{1-4})$-alkylgruppe bedeutet und/oder einer von $R^2$, $R^3$ und $R^4$ eine Alkylgruppe bedeutet und jede der anderen Gruppen die in Anspruch 1 gegebenen Definitionen besitzen, oder ein Salz oder geschütztes Derivat davon hydriert wird und anschließend irgendeine vorhandene Schutzgruppe entfernt wird; oder
(E) eine Schutzgruppe oder mehrere Schutzgruppen von einer geschützten Form einer Verbindung der Formel (I) entfernt wird bzw. werden;

und wenn eine Verbindung der Formel (I) als Gemisch den Enantiomeren erhalten wird, gegebenenfalls das Gemisch unter Herstellung des gewünschten Enantiomeren gespalten wird;

und/oder wenn eine Verbindung der Formel (I) in Form der freien Base erhalten wird, gegebenenfalls die freie Base in ein Salz überführt wird.

7. Verfahren nach Anspruch 6 (A), dadurch **gekennzeichnet**, daß Y eine Alkenylgruppe $=CH_2$ oder eine Gruppe der Formel $CH_2Z$ bedeutet, worin Z ein leicht ersetzbares Atom oder Gruppe bedeutet.

8. Pharmazeutisches Präparat, dadurch **gekennzeichnet**, daß es mindestens eine Verbindung der allgemeinen Formel (I), definiert nach einem der Ansprüche 1 bis 5, oder ein physiologisch annehmbares Salz oder Solvat davon, zusammen mit mindestens einem physiologisch annehmbaren Träger oder Verdünnungsmittel, enthält.